# DEMANDE DE BREVET EUROPEEN

(11) **EP 4 173 794 A1**
(43) Date de publication de la demande: **03.05.2023**
(21) Numéro de dépôt: 22204303.6
(22) Date de dépôt: 28.10.2022
(51) Int. Cl.: B29C 39/10, B29C 39/34, B29C 39/02, G09B 19/00, A61F 2/54, A45D 29/00, A45D 31/00, G09B 23/30, B29C 39/36

(54) **PROCÉDÉ DE FABRICATION D'UN MODÈLE D'AU MOINS UNE PORTION DE MAIN, MOULE PARTICULIÈREMENT DESTINÉ À LA MISE EN OEUVRE D'UN TEL PROCÉDÉ, ET KIT D'ENTRAÎNEMENT COMPRENANT AU MOINS UN MODÈLE OBTENU PAR UN TEL PROCÉDÉ DE FABRICATION**

(30) Priorité: 29.10.2021 FR 2111535
(71) Demandeur: Benard, Fabrice, 1050-218 Lisbonne (PT)
(72) Inventeur: Benard, Fabrice, 1050-218 Lisbonne (PT)
(74) Mandataire: Fidal Innovation

(57) **Abrégé**

Procédé de fabrication d'un modèle (1) de membre comprenant au moins une étape de moulage dans un moule, le moule comprenant une empreinte du modèle et au moins une capsule de moulage d'ongle,
de sorte que le modèle (1) correspond à l'empreinte du moule et comprend au moins une fente (3) correspondant à la capsule de moulage d'ongle et qui est destinée à recevoir une capsule (14) d'ongle.

## Description

### DOMAINE DE L'INVENTION

La présente invention se rapporte au domaine des soins de beauté des mains et des ongles, et plus précisément au domaine de l'entraînement de tels soins.

Encore plus précisément, l'invention se rapporte à un modèle pour l'entraînement aux soins de la beauté des mains et des ongles.

### ARRIÈRE-PLAN TECHNOLOGIQUE

Il convient de distinguer les prothèses et les modèles. En effet, une prothèse remplace un membre manquant, et présente ainsi un certain nombre de propriétés biocompatibles. Dans le cas d'une main, la prothèse peut également présenter des fonctions permettant de reproduire les mouvements d'une main. A l'inverse, un modèle n'a pas pour vocation de remplacer un membre manquant. Un modèle présente une apparence, et éventuellement une texture, proche du membre qu'il cherche à imiter. Ses fonctions sont plus simples que celles d'une prothèse.

La présente invention s'intéresse exclusivement aux modèles, et plus particulièrement aux modèles de main pour l'entraînement aux soins de beauté des mains et des ongles.

Les soins de beauté des mains et des ongles sont réalisés minutieusement pas des professionnels, appelés par exemple esthéticiens, dans des instituts ou à domicile. Différentes techniques sont appliquées pour une mise en beauté d'une main.

Les ongles des mains font l'objet d'une attention particulière. En effet, les ongles peuvent par exemple être limés, peints, ou recouvert avec ce qui est communément appelés des faux-ongles ou capsules, qui peuvent également être à leur tour peints. La peinture (gel, vernis, etc...) des ongles, ou des faux-ongles, qui peut être accompagnée de la pose d'accessoires collés sur les ongles, peut s'avérer complexe. On parle alors de « nail art ».

La technique du nail art requiert de la part du professionnel une grande dextérité afin notamment de poser la peinture avec précision. Beaucoup de facteurs doivent être pris en compte par le professionnel. Par exemple, la texture et la couleur de la peau, mais aussi la tension dans la main du client, peuvent influencer le rendu esthétique recherché par le professionnel.

Ainsi, il est préférable pour les professionnels de s'entraîner sur des modèles de mains avant de passer en situation réelle. Plus le modèle est proche de la réalité, plus le rendu final sur le client sera proche de celui recherché par le professionnel. Ces modèles comprennent des faux-ongles, aussi appelés capsules ou « tips », fixés sur le modèle par exemple à l'aide d'un adhésif, et éventuellement en étant insérées dans une fente du modèle.

Il existe donc des modèles de main dédiés à l'entraînement des professionnels.

Un modèle courant consiste en une main, formée d'un assemblage de pièces articulées les unes aux autres afin de reproduire les articulations d'une main réelle. L'extrémité des doigts est munie d'une fente pour y insérer une capsule de faux ongle pour l'entraînement. Le document JP3202285 décrit un exemple d'un tel modèle de main. Un tel modèle est toutefois peu esthétique, visuellement éloigné d'une main réelle, les articulations entre les pièces étant visibles. L'évaluation esthétique d'un soin de beauté d'une main peut être alors difficile sur une telle main.

Il existe donc des modèles de main cherchant à se rapprocher davantage du visuel d'une main réelle. Le document KR200202191 décrit un exemple d'un modèle de main qui comprend une enveloppe en résine remplie d'une mousse et de tiges en aluminium. Le modèle de main forme des doigts munis d'encoches pour des capsules. Une capsule est fixée dans chaque encoche à l'aide d'un adhésif. L'utilisation de la mousse notamment ne permet pas de reproduire fidèlement la souplesse d'une main réelle. En outre, le collage des capsules est fastidieux. Le modèle étant destiné à l'entrainement, il est souhaitable de pouvoir l'utiliser plusieurs fois, et donc de pouvoir retirer les faux-ongles pour en placer des nouveaux. Le collage implique une étape fastidieuse de nettoyage pour retirer l'adhésif.

Il existe également des modèles en silicone. Le silicone permet d'avoir un aspect, une texture et une souplesse qui se rapprochent des ceux d'une main réelle. Il se prête également bien au moulage. Ainsi, de manière connue, un modèle de silicone peut être obtenu par moulage. Des fentes sont découpées à l'extrémité des doigts pour pouvoir y glisser des capsules.

La technique classique du moulage pour une main consiste à couler du silicone dans un moule en au moins deux parties, voire trois parties, le démoulage étant obtenu par la séparation des parties du moule. Cette technique permet de démouler facilement le silicone. Toutefois, un plan de joint apparaît alors sur le modèle. Une étape d'élimination du plan de joint doit alors être prévue, ce qui peut dégrader l'aspect du modèle ainsi qu'augmenter les coûts de fabrication.

En outre, les fentes obtenues par découpage ne permettent pas de retenir suffisamment les capsules uniquement par friction, car elles ne sont pas parfaitement ajustées aux dimensions des capsules. Ainsi, afin d'éviter que les capsules ne bougent pendant l'entraînement du professionnel sur le modèle, un adhésif est ajouté entre les capsules et le modèle, avec les inconvénients précités.

Il existe donc un besoin pour un nouveau modèle de main et un nouveau procédé de fabrication d'un tel modèle surmontant en particulier les inconvénients mentionnés de l'art antérieur.

### RÉSUMÉ DE L'INVENTION

Ainsi, selon un premier aspect, l'invention se rapporte à un procédé de fabrication d'un modèle d'au moins une portion de membre, tels qu'une main ou un pied. Le modèle est destiné notamment à l'entraînement aux soins des membres et/ou des ongles. Le procédé comprend au moins une étape de moulage dans un moule qui comprend une empreinte du modèle et qui comprend de plus au moins une capsule de moulage d'ongle. La capsule de moulage d'ongle comprend au moins une portion en prise dans le moule et au moins une portion en saillie à l'intérieur du moule. L'étape de moulage comprend alors notamment :
- le remplissage au moins en partie du moule d'un matériau pour former le modèle ;
- la prise au moins partielle du matériau dans le moule et autour de la portion en saillie de la capsule de moulage ;
- l'extraction du modèle hors du moule.

Ainsi, le modèle correspond à l'empreinte du moule et comprend au moins une fente correspondant à la capsule de moulage d'ongle et qui est destinée à recevoir une capsule d'ongle.

La fente ainsi formée par moulage n'a pas besoin d'être découpée dans une étape ultérieure, réduisant le temps de fabrication du modèle et donc les coûts. En outre, en utilisant une capsule d'ongle adéquate pour l'entraînement, la fente est parfaitement adaptée à la capsule d'ongle pour la maintenir en place dans la fente pendant l'entrainement d'un professionnel sans avoir à apporter de matière de type colle ou autre adhésif. Ainsi, aucun nettoyage n'est non plus requis lorsque le professionnel veut changer la capsule d'ongle. Le modèle obtenu par moulage est monobloc, sans partie articulée par des liaisons fragiles et couteuses à mettre en place.

Selon différents aspects, il est possible de prévoir l'une et/ou l'autre des caractéristiques ci-dessous prises seules ou en combinaison.

Selon un mode de réalisation, le moule peut être fabriqué à partir d'un modèle primaire de l'au moins une portion de membre, le procédé comprenant :
- la fixation de la au moins une capsule de moulage d'ongle sur le modèle primaire, de sorte que la capsule de moulage d'ongle comprend au moins une portion libre, en saillie sur le modèle primaire ;
- l'enrobage du modèle primaire et de la capsule de moulage d'ongle dans un matériau adapté à former un moule ;
- la prise de la au moins une portion libre de la capsule de moulage d'ongle dans le matériau adapté à former le moule ;
- l'extraction du modèle primaire hors du moule, la capsule de moulage restant en place dans le moule.

On obtient ainsi simplement et rapidement un moule avec une capsule de moulage disponible pour former une fente dans le modèle. La portion libre devient alors la portion en prise dans le moule. La capsule de moulage peut, sans contrainte supplémentaire, être choisie pour que sa portion en saillie correspondante à la portion de la capsule d'ongle qui sera insérée dans la fente.

La capsule de moulage peut alors comprendre sur la au moins une portion libre au moins une ouverture pour la prise avec le matériau adapté à former le moule. Ainsi, le risque que la capsule de moulage ne soit retiré au moment de l'extraction du modèle primaire hors du moule est limité, voire quasiment nul.

Selon un mode de réalisation, le moule est monobloc, et comprend une ouverture, qui est de préférence unique, pour le remplissage du moule et pour l'extraction du modèle. Ainsi, le modèle est dénué de bavure de plan de joint, pour un aspect esthétique agréable, sans étape supplémentaire pour masquer les bavures des plans de joints

A cet effet, il peut être prévu un système de préhension fixé sur le modèle. Le procédé comprend alors, avant l'extraction du modèle hors du moule, la mise en place du système de préhension sur le modèle et la prise du système de préhension dans le matériau pour le modèle. L'extraction du modèle hors du moule peut alors comprendre la coopération entre le système de préhension en prise avec le matériau du modèle dans le moule et un dispositif d'extraction. L'extraction s'en trouve facilitée, sans dégrader le modèle.

Selon un mode de réalisation, après l'extraction du modèle hors du moule, le procédé peut comprendre le retrait complet du système de préhension hors du modèle.

Selon un mode de réalisation, le système de préhension comprend un support pour un aimant, et le procédé peut comprendre après l'extraction du modèle hors du moule le retrait d'une partie seulement du système de préhension, le support pour l'aimant restant en place sur le modèle.

Selon un mode de réalisation, le modèle peut comprendre au moins un doigt d'une main. En variante, le modèle peut comprendre une main complète. Le moule n'est pas limité dans la représentation du modèle, e manière à laisser le professionnel choisir le modèle selon ses préférences et habitudes.

Selon un mode de réalisation, le matériau du modèle peut comprendre du silicone, qui permet d'obtenir un modèle dont la texture et la souplesse se rapproche de celles d'un membre humain. Le silicone permet en outre d'être coloré, afin de se rapprocher d'une couleur naturelle de peau notamment.

Selon un deuxième aspect, l'invention propose un moule pour la fabrication d'un modèle d'au moins une partie de membre, tels qu'une main ou un pied. Le moule comprend une empreinte de la au moins une partie de membre et au moins une capsule de moulage d'ongle. La capsule de moulage d'ongle peut comprendre au moins une portion en prise dans le moule et au moins une portion en saillie à l'intérieur du moule. Le moule peut alors être particulièrement destiné à la mise en oeuvre du procédé de fabrication tel que présenté ci-dessus.

Selon un troisième aspect, l'invention propose un kit pour l'entraînement aux soins d'un membre tel qu'un pied ou une main et/ou des ongles. Le kit comprend au moins un modèle obtenu par la mise en oeuvre du procédé de fabrication tel que présenté ci-dessus. Le modèle est alors muni d'au moins une fente et le kit peut comprendre au moins une capsule d'ongles de forme et de dimension ajustées à celles de la fente.

### BRÈVE DESCRIPTION DES DESSINS

Des modes de réalisation de l'invention seront décrits ci-dessous par référence aux dessins, décrits brièvement ci-dessous :
[Fig. 1] représente de manière schématique un modèle d'une main complète pour l'entrainement aux soins de la beauté des mains et/ou des ongles.

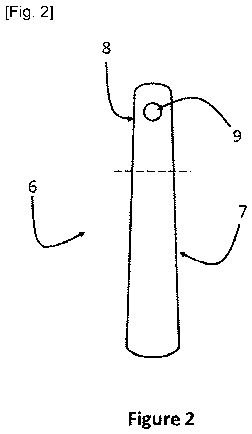

[Fig. 2] représente une capsule de moulage.

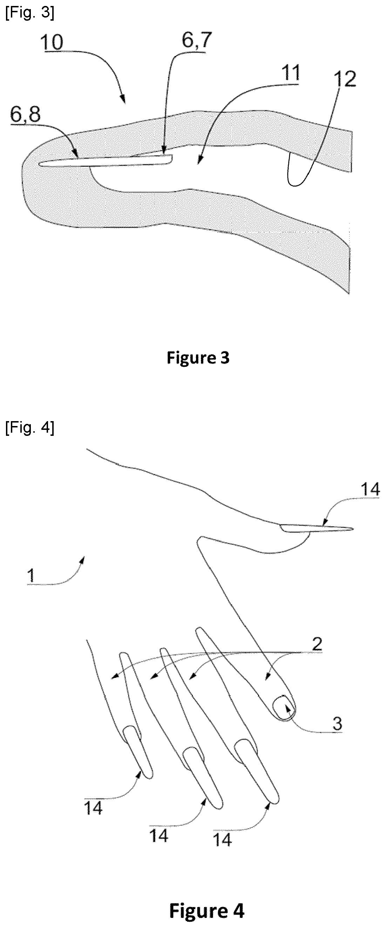

[Fig. 3] représente une vue en coupe transversale du moule de la figure 3.
[Fig. 4] représente une vue d'une partie du modèle de la figure 1.

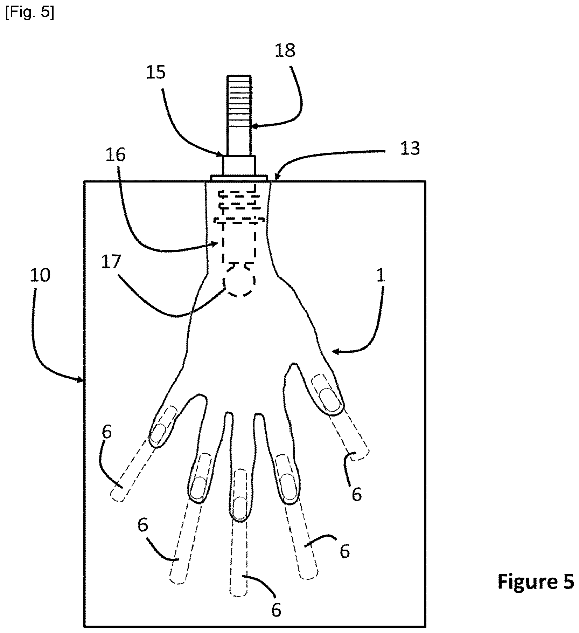

[Fig. 5] représente de manière schématique le modèle de la figure 1 en cours de fabrication dans un moule, le modèle comprenant un système de préhension selon un premier mode de réalisation.

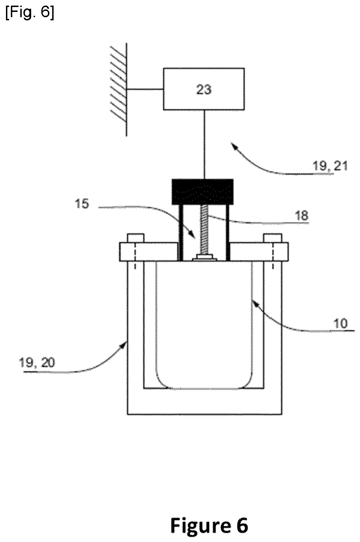

[Fig. 6] représente de manière schématique l'étape d'extraction du modèle hors du moule de la figure 5 à l'aide d'un dispositif d'extraction.

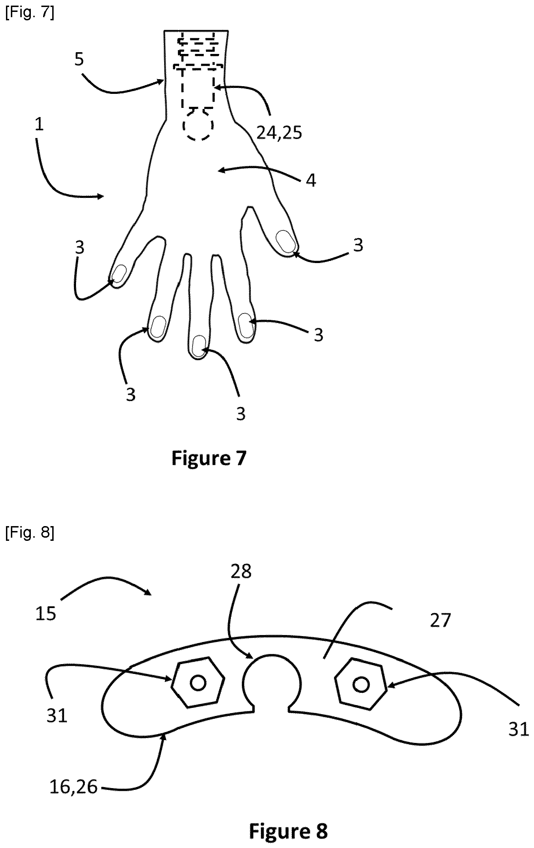

[Fig. 7] représente de manière schématique le modèle de la figure 6 après l'étape d'extraction.
[Fig. 8] représente un support d'un système de préhension selon un deuxième mode de réalisation.

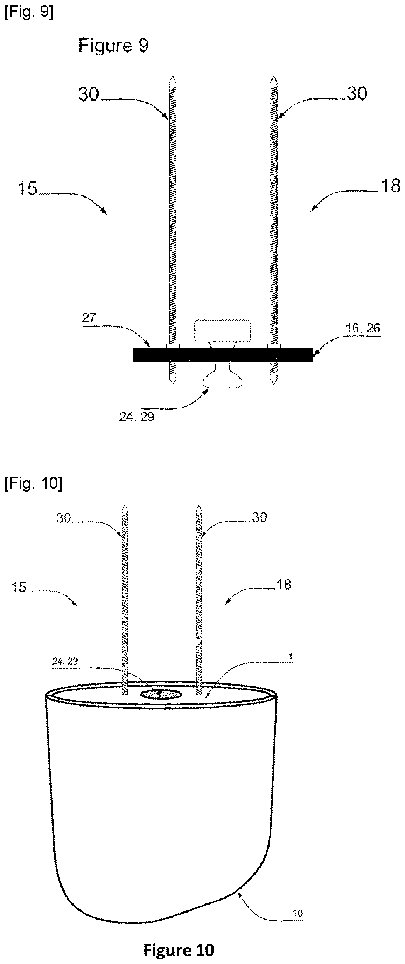

[Fig. 9] représente le système de préhension selon le deuxième mode de réalisation.
[Fig. 10] représente le système de préhension selon le deuxième mode de réalisation mis en place sur un moule.

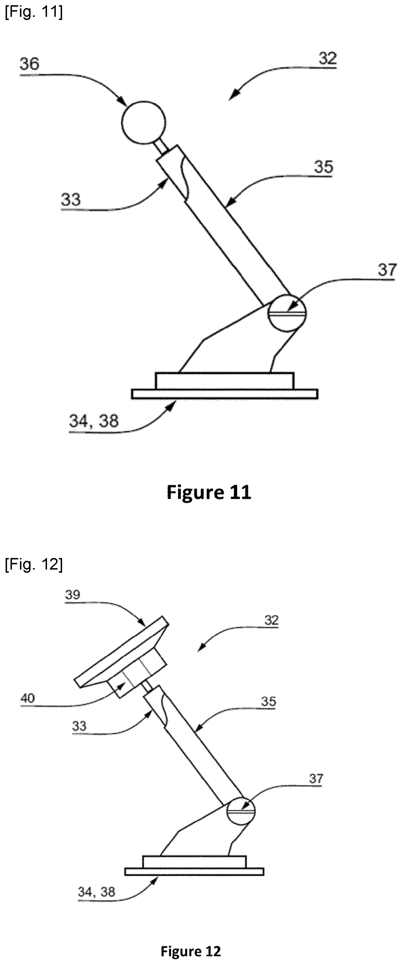

[Fig. 11] représente un présentoir selon un premier type.
[Fig. 12] représente un présentoir selon un second type.

Sur les dessins, des références identiques désignent des objets identiques ou similaires.

### DESCRIPTION DÉTAILLÉE

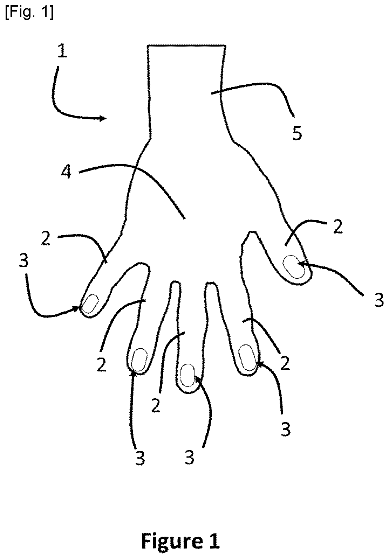

Sur la figure 1, il est représenté de manière schématique un modèle **1** d'une portion d'un membre pour l'entraînement de professionnel aux soins des mains et/ou des ongles. Par membre, on désigne ici un membre supérieur ou inférieur d'un être humain, et plus précisément une main ou un pied. De manière générale, selon l'invention, le modèle peut concerner tout ou partie d'un membre, par exemple un doigt ou un orteil, ou plusieurs doigts reliés par une portion de paume, ou une main ou un pied complet jusqu'au poignet ou à la cheville voire au-delà.

Selon l'exemple de la figure 1, le modèle comprend une main complète, avec les cinq doigts **2.** L'extrémité de chaque doigt 2 est munie d'une fente **3** pour pouvoir y glisser une capsule d'ongle.

Le modèle 1 comprend également une paume **4,** et un poignet **5.**

Les doigts 2, la paume 4 et le poignet 5 forment un modèle 1 monobloc, c'est-à-dire que le modèle 1 forme une seule et unique pièce, sans assemblage ni liaison entre des parties du modèle 1.

La dimension de chaque fente 3 est parfaitement ajustée à celle de la capsule d'ongle correspondante. A cet effet, il a été mis au point un procédé de fabrication permettant d'obtenir, par moulage, le modèle 1 muni des fentes 3, grâce notamment à un moule comprenant des capsules 6 de moulage d'ongles intégrées au moule.

Il va maintenant être décrit un mode de réalisation pour la fabrication du modèle 1 muni des fentes 3.

Selon un mode de réalisation, le moule pour mouler le modèle 1 est obtenu à partir d'un modèle primaire de main.

Plus précisément, le modèle primaire de main est mis au point dans une étape à partir d'une prise d'empreinte d'une main réelle de manière connue, par exemple à l'aide de silicone spécifique pour la prise d'empreinte sur la peau, permettant de prendre une empreinte avec une grande précision. L'empreinte ainsi obtenue est recouverte d'une fine couche de silicone, formant un gant creux. Le gant est rempli en partie d'un matériau adapté, par exemple du silicone. Plus précisément, les doigts du gant sont remplis, par exemple jusqu'à environ la première phalange, et en tout cas d'une quantité suffisante de silicone pour pouvoir ensuite répartir le silicone à l'intérieur du gant de manière à former une couche. Si besoin, plusieurs couches superposées peuvent ainsi être formées, jusqu'à ce que, après séchage, le gant présente une rigidité suffisante. Des capsules 6 de moulage d'ongles sont en outre insérées dans le silicone du gant. Par exemple, une fois le gant en silicone suffisamment durci, mais toujours creux, l'ongle de chaque doigt du gant est découpé et un logement est réalisé pour pouvoir y insérer une capsule 6 de moulage, sur chaque doigt.

Une capsule 6 de moulage est un élément en matériau rigide, en plastique par exemple. Elle comprend une première portion **7** destinée à être insérée dans le modèle primaire, et une deuxième portion **8,** libre par rapport au modèle primaire. Au moins la première portion 7 est similaire à la portion d'une capsule d'ongle qui sera insérée sur le modèle 1 de main pour l'entraînement d'un professionnel. La deuxième portion 8 peut comprendre au moins un orifice **9,** pour des raisons qui seront exposées plus loin. La première portion 7 présente une longueur correspondant à celle attendue pour l'insertion d'une capsule d'ongle sur le modèle. La longueur d'insertion est au moins de 1cm, de préférence au moins de 2 cm. La deuxième portion 8 présente une longueur suffisante pour assurer, comme cela sera vu plus loin, une prise suffisante dans le moule.

Une fois les capsules 6 de moulage insérées sur le gant, ce dernier est durci afin d'atteindre une rigidité suffisante pour la suite des opérations. Par exemple, il peut être rempli totalement de silicone, ou en tout cas sur une épaisseur suffisante pour atteindre la rigidité requise, formant le modèle primaire de main équipé des capsules 6 de moulage.

Le moule 10 peut alors être fabriqué à partir du modèle primaire et des capsules 6 de moulage.

Selon un mode de réalisation, le moule 10 est fabriqué par enrobage du modèle primaire et des capsules 6 de moulage, dans un matériau adapté à former un moule. Il s'agit par exemple d'une résine telle qu'une résine de polyuréthane. L'enrobage peut être réalisé par rotomoulage. L'enrobage recouvre ainsi le modèle primaire et la deuxième portion 8 des capsules 6 de moulage, et pénètre à travers les orifices 9 des capsules 6 de moulage, assurant la prise dans le matériau du moule des capsules 6 de moulage. Une étape de séchage et/ou de cuisson peut être mise en œuvre en fonction du matériau du moule 10.

Le modèle primaire est alors extrait du moule 10 ainsi obtenu. Les capsules 6 de moulage en prise avec le moule 10 se détachent du modèle primaire et demeurent fixées au moule 10.

Le moule 10 ainsi obtenu comprend alors une empreinte 11 en creux représentant la main et délimitée par une paroi **12** intérieure du moule 10, et les capsules 6 de moulage. Plus précisément, la première portion 7 des capsules 6 de moulage se retrouve en saillie à l'intérieur du moule 10, tandis que la deuxième portion 8 est en prise dans le moule.

Le moule 10 est monobloc, c'est-à-dire qu'il est formé d'un seul tenant, sans élément admettant un mouvement les uns par rapport aux autres. Il comprend une ouverture 13, par exemple d'un côté opposé à celui des capsules 6 de moulage, tel qu'au niveau du poignet.

### Le moule ainsi obtenu est alors utilisé pour la fabrication des modèles.

Il va maintenant être décrit un exemple d'un procédé de fabrication d'un modèle 1 de main à partir du moule 10. La matériau utilisé pour réaliser le modèle est par exemple du silicone. Le silicone présente notamment l'avantage de pouvoir être coloré, de pouvoir être moulé, d'avoir une texture qui s'approche de celle de la peau humaine, et qui en outre présente une souplesse et une élasticité proches de celle de la peau humaine.

Selon un mode de réalisation, le moule 10 peut être préalablement chauffé dans un four.

Le matériau prévu pour le modèle est versé par l'ouverture 13, par exemple sous forme liquide, dans le moule 10, chaud le cas échéant.

Selon un mode de réalisation, le moule 10 est rempli de ce matériau jusqu'à un niveau déterminé en une unique fois, de la quantité requise pour former le modèle. Avant de couler le matériau dans le moule 10, il peut être préalablement placé sous vide pour dégazer. Puis, le moule 10 rempli peut être posé sur une table vibrante pendant que le matériau est coulé et/ou juste après, permettant limiter les risques de formation de bulles d'air à l'intérieur du moule 10, et dans une position sensiblement verticale dans laquelle l'ouverture 13 est orientée vers le haut. Le matériau du modèle, sous forme liquide, prend la forme de l'empreinte 11 dans le moule et enrobe la première portion 7 des capsules 6 de moulage.

Selon un autre mode de réalisation, le matériau du modèle est versé dans le moule 10, sans toutefois remplir entièrement le moule 10. Là encore, après mise sous vide du matériau du modèle, une table vibrante, avec le moule 10 dans une position verticale, peut être mise en œuvre pendant que le matériau est coulé dans le moule 10 et/ou juste après. Par exemple, le moule 10 est rempli jusqu'au-dessus de l'articulation des doigts de l'empreinte 11 dans le moule 10. Le moule 10 est alors mis en rotation de manière à ce que le matériau du modèle, encore sous forme liquide, se répartit sur et recouvre la paroi 12 intérieure de l'empreinte 11, formant une couche de matériau du modèle sur la paroi 12 intérieure. Selon ce mode de réalisation, il est recommandé, mais pas nécessaire, d'utiliser le moule 10 préalablement chauffé, comme vu précédemment, de sorte que le matériau du modèle peut se rigidifier plus rapidement qu'avec un moule froid, en quelques secondes ou quelques minutes. On obtient alors une couche figée d'environ 1 à 3mm. Eventuellement, plusieurs couches du matériau du modèle peuvent ainsi être superposées sur la paroi 12 intérieure du moule 10, jusqu'à l'obtention d'une épaisseur déterminée. Ace stade, il peut être en outre prévu que la première portion 7 des capsules 6 de moulage soit immergée dans le matériau du modèle. Le moule 10 avec la couche de matériau du modèle peut éventuellement être passé dans un four afin d'accélérer la prise du matériau du modèle.

Une armature peut alors être insérée dans le moule 10. L'armature peut comprendre par exemple un ensemble de tiges, chaque tige étant placée dans un doigt du moule. Chaque tige est déformable élastiquement manuellement en flexion, c'est-à-dire quelle prendre une position en étant pliée sous un effort exercé manuellement, et conserver cette position lorsque l'effort est retiré. Par exemple, chaque tige comprend un cœur en métal et une gaine en matière plastique. Les extrémités de chaque tige peut comprendre un bouchon arrondi, par exemple en plastique, pour éviter de blesser le matériau du modèle une fois la tige placée dans le moule 10.

Une fois les tiges positionnées dans le moule 10, ce dernier peut être rempli du matériau de modèle jusqu'au niveau déterminé, avec la quantité prévue pour former le modèle complet. Là encore, la table vibrante peut être utilisée.

Le moule 10 rempli est alors conservé dans une position verticale, l'ouverture 13 dirigée vers le haut, jusqu'à la prise du matériau de modèle. Par exemple, dans le cas où le matériau du modèle est du silicone, le moule 10 rempli peut être laissé à température ambiante sur une durée de l'ordre de 30 à 40 minutes. Puis le moule 10 peut être placé dans une étuve pour le chauffer modérément, par exemple à 40°C environ pendant 1h. En variante, le moule 10 peut être laissé à température ambiante jusqu'à obtenir le durcissement total du matériau dans le moule.

Puis, le modèle 1 est extrait du moule 10 en exerçant un effort de traction sur le modèle 1 par l'ouverture 13 du moule 10. Le modèle 1 se détache alors de la paroi 12 intérieure du moule 12 et des capsules 6 de moulage pour sortir par l'ouverture 12. Le modèle 1 correspond ainsi à l'empreinte 11 du moule, et comprend des fentes 3 formées par les capsules 6 de moulage.

Le modèle 1 ainsi obtenu par le moule monobloc ne comprend pas de plan de joint, et les fentes 3 sont déjà formées, de sorte qu'il ne requiert pas d'étape supplémentaire de fabrication. Eventuellement, le modèle 1 peut être coloré pour approcher davantage visuellement d'une main réelle.

Des capsules **14** d'ongles dont les dimensions correspondent à celles des capsules 6 de moulage peuvent être fournies. Plus généralement, chaque capsule 14 d'ongle est dimensionnée pour correspondre à un doigt 2, et comprend une portion destinée à être insérée dans une fente 3 du doigt 2 correspondant du modèle 1 qui reprend les dimensions de la première portion 7 de la capsule 6 de moulage du doigt en question. Ainsi, la capsule 14 d'ongle est parfaitement ajustée à la fente 3 du doigt correspondant, et peut être retenue sur le modèle 1 par la seule force de friction. Aucun adhésif supplémentaire ni dispositif de liaison supplémentaire n'est nécessaire. Eventuellement, lorsque le matériau du modèle est du silicone notamment, lors de la mise en place des capsules 14 d'ongles, le silicone peut être légèrement écarté manuellement autour de la fente 3, pour faciliter l'insertion de la capsule 14. Puis, grâce à son élasticité, le silicone reprend sa forme initiale, venant s'ajuster exactement autour de la capsule 14 pour la maintenir.

Selon un mode de réalisation, et de manière indépendante à ce qui précède concernant la formation des fentes 3, afin de faciliter la manipulation du moule 10, et notamment l'étape d'extraction, un système **15** de préhension est fixé sur le modèle 1 en cours de fabrication, dans le moule 10. Le système 15 de préhension comprend une portion **16** de mise en prise avec le modèle 1 et une portion **18** de de préhension.

Selon un premier mode de réalisation, le système 15 de préhension se présente sous la forme d'une tige. La portion 16 de mise en prise est par exemple de forme tubulaire cannelée, et peut être munie à son extrémité d'une boule **17.** La portion 18 de préhension est par exemple filetée. Les deux portions 16, 18 sont dans le prolongement l'une de l'autre.

Selon un mode de réalisation, la portion 16 de mise en prise, cannelée, du système 15 de préhension est plongée dans le moule 10 pendant le remplissage avec le matériau du modèle, de sorte que lors de la prise du matériau dans le moule, la portion 16 cannelée est emprisonnée dans le matériau du modèle. La portion 18 filetée émerge alors par l'ouverture 13 du moule 10.

Les cannelures de la portion 16 cannelée permettent une bonne tenue du système 15 de préhension dans le modèle 1.

Le système 15 de préhension peut alors être utilisé à plusieurs étapes du procédé, lorsque le moule 1 rempli doit être manipulé.

Notamment, le système 15 de préhension trouve une application particulière pour l'extraction du modèle 1 hors du moule, indépendamment de la formation des fentes 3 par les capsules 6 de moulage. En effet, le moule 10 étant monobloc, le modèle 1 est extrait du moule 10 en exerçant un effort de traction sur le modèle 1. A cet effet, un dispositif **19** d'extraction est mis en coopération avec le système 15 de préhension, et plus précisément avec la portion 18 de préhension.

A cet effet, le dispositif 19 d'extraction peut comprendre un socle **20** de retenue du moule 10 et un système **21** de traction, par exemple de type treuil. Le socle 20 peut comprendre un étau serrant le moule 10, ou une barre venant en appui contre le moule 10, autour de l'ouverture 13. La portion 18 filetée est fixée, à l'aide d'un écrou **22** par exemple, sur le système 21 de traction. Un dispositif **23** de commande du système 21 de traction permet d'exercer sur le dispositif 15 de préhension, par l'intermédiaire de l'écrou 22, un effort de traction emportant le modèle 1 hors du moule 10, la portion 16 cannelée assurant la retenue du système 15 de préhension sur le modèle pendant l'application de l'effort de traction.

Une fois le modèle 1 extrait du moule 10, le système 15 de préhension peut être retiré du modèle 1.

Le modèle 1 peut en outre comprendre un dispositif **24** d'assemblage avec un présentoir permettant au professionnel d'utiliser le modèle 1 pour l'entraînement.

Selon un mode de réalisation, le dispositif 24 de coopération comprend une empreinte **25** formée par le retrait du système 15 de préhension décrit ci-dessus. Par exemple, lorsque le matériau utilisé est du silicone, en écartant le matériau autour de la portion 16 cannelée, le dispositif 15 de préhension peut être retiré manuellement. L'empreinte 25 du système 15 de préhension est alors formée dans le modèle 1, sensiblement au moins en partie au niveau du poignet 5. Comme cela sera vu plus loin, cette empreinte 25 permet d'assembler le modèle 1 sur un présentoir.

Selon un autre mode de réalisation du système 15 de préhension, ce dernier sert également à la fixation sur le modèle 1 d'un aimant. Ce mode de réalisation est particulièrement adapté au cas dans lequel le modèle représente une portion d'un membre, et notamment lorsque le modèle concerne un doigt ou une paume de main comprenant quatre doigts.

Le terme « aimant » désigne ici tout aimant permanent ou induit.

Plus précisément, selon ce deuxième mode de réalisation, la portion 16 de mise en prise comprend un support **26,** par exemple sous forme de plaque. La plaque 26 est conformée et dimensionnée de manière à pouvoir être placée dans le moule 10 en présentant une face **27** supérieure tournée vers l'ouverture 13 du moule 10. Elle est par exemple fabriquée par une technique de fabrication additive. La plaque 26 peut être munie d'un logement 28 pour un aimant. Le logement 28 est par exemple une ouverture sur la plaque 26 dans laquelle un aimant **29** est inséré pour y être retenu et affleurer ou émerger au-delà de la face 27 supérieure. La portion 18 de préhension du système 15 comprend alors au moins une, et de préférence deux, tiges filetées **30,** qui viennent s'insérer dans deux ouvertures **31** de la plaque 26. Les ouvertures 31 sont par exemple taraudées, ou un des écrous peuvent être utilisés, du côté opposé à celui la surface 27 supérieure de la plaque 26, pour maintenir les tiges 30 sur la plaque 26.

Ainsi, selon le deuxième mode de réalisation, la plaque 26 munie de l'aimant 29 et des tiges 30 filetées est placée dans le moule 10 pendant le remplissage avec le matériau du modèle, de sorte que le matériau vienne en prise avec la plaque 26 tout en laissant au moins une partie de l'aimant émergeant, et accessible par l'ouverture 13 du moule 10. Les tiges 30 filetées sont également accessibles hors du moule 10. De préférence, la plaque 26 est totalement immergée dans le matériau de modèle afin d'être invisible et d'assurer une bonne tenue de la plaque 26 sur le modèle 1.

Les tiges 31 filetées peuvent alors être utilisées d'une manière similaire à ce qui a été décrit pour le premier mode de réalisation du système 15 de préhension en les fixant à l'aide d'écrou à un dispositif 29 d'extraction pour extraire le modèle 1 hors du moule 10.

Une fois le modèle 1 extrait hors du moule, les tiges 30 filetées peuvent être démontées de la plaque 26 par dévissage. La plaque 26 et l'aimant 29 restent alors en place dans le modèle 1. La plaque 26 est de préférence invisible, tandis que l'aimant est visible.

Le dispositif 24 d'assemblage du modèle 1 avec un présentoir comprend alors astucieusement l'aimant 29.

Les dispositifs 15 de préhension et le dispositif 19 d'extraction décrits ci-dessus peuvent également être mis en œuvre sur le modèle primaire afin de l'extraire du moule 10 une fois que le moule 10 est formé et durci.

Le modèle 1 ainsi extrait du moule 10 est ainsi prêt à être utilisé. Il peut par exemple être assemblé avec un présentoir pour être utilisé par un professionnel. Le matériau du modèle tel que du silicone peut être coloré avant la mise dans le moule 10, à l'aide d'une couleur proche d'une couleur de carnation déterminée. En variante ou en combinaison, de manière facultative, il peut être prévu d'apposer une couche de coloration sur le modèle 1 à la sortie du moule 10 afin d'augmenter la ressemblance avec une main réelle, par exemple à l'aide d'un aérographe.

Un présentoir **32** comprend typiquement une portion **33** d'assemblage sur le modèle, et une portion **34** de fixation sur un support. Le support peut être un mur, un sol, un plafond ou un élément de mobilier tel qu'une table, et comprend de manière générale au moins une surface d'assemblage. En variante, le support peut être une plaque mobile posé sur une surface, de sorte qu'il peut être déplacé volontairement par le professionnel tout en procurant un lest suffisant pour ne pas être déplacé malencontreusement pendant une action sur le modèle.

Différentes types de présentoir peuvent être prévus. Nous allons en présenter trois types dans ce qui suit.

Un premier type de présentoir **32** est destiné à être assemblé avec un modèle 1 dont le dispositif 24 d'assemblage comprend l'empreinte 25 du système 15 de préhension selon le premier mode de réalisation. La portion 33 d'assemblage comprend alors une tige **35** dimensionnée et conformée pour correspondre à l'empreinte 24. Notamment, la tige 35 comprend à une extrémité une boule **36** correspondant à la boule 17 du système 15 de préhension du premier mode de réalisation. Selon un mode de réalisation, l'autre extrémité de la tige 35 est connectée à la portion 34 de fixation à l'aide d'une liaison 37 charnière.

La portion 34 de fixation peut comprendre tout dispositif lui permettant d'être fixée sur un support. La fixation est de préférence amovible, afin de pouvoir déplacer le présentoir 32. La portion 34 de fixation peut alors comprendre par exemple une pince ou un système de vissage. Selon un mode de réalisation particulier, la portion 34 de fixation comprend une ventouse **38** lui permettant de se fixer de manière amovible sur une surface du support, indépendamment de l'inclinaison de la surface en question. Dans le cas de la ventouse 38, le support présente une surface lisse. Il s'agit par exemple d'une plaque de marbre, assez lourde pour procurer le lest requis tout en permettant d'être déplacée par I professionnel.

Ainsi, le présentoir 32 peut facilement être fixé sur un support à l'aide de la ventouse 38. Le modèle 1 est assemblé au présentoir 32 en insérant la tige 35 dans l'empreinte 25, jusqu'à ce que la boule 36 du présentoir 32 vienne dans l'empreinte de la boule 17 du système 15 de préhension. Le modèle 1 est ainsi tenu en porte-à-faux sur le présentoir par la tige 35. Lorsque le modèle 1 est une main, la tige 35 est insérée au niveau du poignet 5 du modèle, et de préférence ne s'étend pas jusqu'à la paume 4, afin de conserver une souplesse du reste du modèle, de manière à laisser plus de liberté au professionnel pour positionner le modèle selon ses besoins. Au moins un degré de liberté en rotation autour de la tige 35 est permise pour le modèle 1. Le professionnel peut alors orienter le modèle 1 dans une position adéquate grâce à la fois à la liaison charnière 37 du présentoir 32, à la rotation autour de la tige 35, et à l'élasticité du modèle 1 lui-même, assisté de l'armature le cas échéant. Lorsque le support est mobile, il peut également offrir des degrés de liberté supplémentaires.

Ce premier type de présentoir 32 est particulièrement adapté au modèle 1 de main complète.

Un deuxième type de présentoir 32 est destiné à être assemblé avec un modèle 1 dont le dispositif 24 d'assemblage comprend l'aimant 29. La portion 33 d'assemblage comprend alors une base **39** comprenant un élément aimanté, par exemple métallique, destiné à permettre la fixation par aimantation de l'aimant 29 du modèle 1. La base 39 est montée à une extrémité d'une tige **35,** dont l'autre extrémité est connectée à la portion 34 de fixation à l'aide d'une liaison **37** charnière. La portion 34 de fixation est similaire à celle du présentoir du premier type, et comprend notamment une ventouse 38.

Le modèle 1 est alors assemblé au présentoir 32 en posant l'aimant 29 contre la base 39 du présentoir 32. Le modèle 1 peut là encore être orienté par le professionnel en fonction de ses besoins grâce notamment à la liaison charnière 37, à la rotation du modèle sur la base 39, l'aimantation laissant un degré de liberté en rotation, et à l'élasticité du modèle 1 lui-même, assisté de l'armature le cas échéant.

Le présentoir 32 du deuxième type trouve une application particulière pour l'assemblage avec un modèle 1 d'une partie d'une main. Selon un mode de réalisation, la surface métallique de la base 39 peut être suffisamment étendue pour y assembler plusieurs modèles 1, chaque modèle étant par exemple une portion d'une main. Par exemple, chaque modèle 1 peut correspondre à un doigt d'une main. Le présentoir 32 selon le deuxième type peut donc permettre à un professionnel de choisir les doigts sur lesquels s'entraîner, voire de reconstituer les cinq doigts de la main.

Selon un troisième type, le présentoir 32 est une combinaison du premier type et du deuxième type présenté ci-dessus. Par exemple, la base 39 du deuxième type est montée de manière amovible sur la tige 37, par-dessus la boule 36 du premier type. A cet effet, la base 39 peut comprendre un système **40** de serrage, par exemple sous forme d'un écrou, permettant de serrer et de desserrer la base 39 sur la tige 35. En retirant la base 39, il est alors possible de passer du deuxième type au premier type de présentoir 32. Inversement, en remontant la base 39 sur la tige 35, on peut passer du premier type au deuxième type de présentoir 32.

Le présentoir 32 facilite le retrait et la mise en place du modèle 1. En effet, il suffit soit de faire glisser le modèle 1 sur la tige 35 ou de le poser sur la base 39 pour le mettre en place sur le modèle. L'opération inverse de retrait est tout aussi simple, de sorte que le modèle, une fois que le professionnel a terminé son entraînement dessus, peut être retiré du présentoir 32 en un unique mouvement. Notamment, il est courant, afin d'obtenir un séchage plus rapide et/ou de meilleure qualité de la peinture sur les faux ongles, de passer le modèle 1 sous un rayonnement UV. En limitant les actions sur le modèle 1 pour le retirer du présentoir 32, alors que le travail du professionnel n'est pas encore sec, le risque d'abîmer le travail du professionnel sur les capsules est également limité.

Ainsi, il peut être fourni un kit comprenant un présentoir 32, un modèle 1 et des capsules 14 d'ongles associées. Le présentoir 32 est par exemple du troisième type, de manière à pouvoir s'adapter aussi bien à un modèle 1 dont dispositif 24 d'assemblage comprend l'empreinte 25 qu'à un modèle 1 dont le dispositif 24 d'assemblage comprend un aimant. La fourniture des capsules 14 à des dimensions ajustées à celles de fentes 3 assure une tenue adéquate des capsules 14 sur le modèle sans élément supplémentaire pour l'entraînement des professionnels des soins de la beauté des mains. L'entraînement du professionnel s'en trouve simplifiée. En effet, d'une part la mise en place des capsules 14 d'ongles se fait par simple insertion, sans avoir à manipuler un élément supplémentaire de type adhésif, et d'autre part le retrait des capsules 14 se fait en exerçant un simple effort de traction sur les capsules 14. Une fois les capsules retirées, le modèle 1 peut de nouveau être utilisé sans avoir à nettoyer des restes d'adhésifs. Le kit étant dépourvu d'adhésif, son coût est réduit.

## Revendications

1. Procédé de fabrication d'un modèle (1) d'au moins une portion de membre, le modèle (1) étant destiné à l'entraînement aux soins des membres et/ou des ongles, le procédé comprenant au moins une étape de moulage dans un moule (10), le moule (10) comprenant au moins une empreinte (11) du modèle et au moins une capsule (6) de moulage d'ongle, la capsule (6) de moulage d'ongle comprenant au moins une portion (8) en prise dans le moule et au moins une portion (7) en saillie à l'intérieur du moule (10), l'étape de moulage comprenant :
- le remplissage au moins en partie du moule (10) d'un matériau pour former le modèle ;
- la prise au moins partielle du matériau dans le moule (10) et autour de la portion (7) en saillie de la capsule (6) de moulage ;
- l'extraction du modèle (1) hors du moule (10),
de sorte que le modèle (1) correspond à l'empreinte (11) du moule (10) et comprend au moins une fente (3) correspondant à la capsule (6) de moulage d'ongle et qui est destinée à recevoir une capsule (14) d'ongle.

2. Procédé selon la revendication 1 dans lequel le moule (10) est fabriqué à partir d'un modèle primaire de l'au moins une portion de membre, le procédé comprenant :
- la fixation de la au moins une capsule (6) de moulage d'ongle sur le modèle primaire, de sorte que la capsule (6) de moulage d'ongle comprend au moins une portion (8) libre, en saillie sur le modèle primaire ;
- l'enrobage du modèle primaire et de la capsule (6) de moulage d'ongle dans un matériau adapté à former un moule (10) ;
- la prise de la au moins une portion (8) libre de la capsule (6) de moulage d'ongle dans le matériau adapté à former le moule (10) ;
- l'extraction du modèle primaire hors du moule (10), la capsule (6) de moulage restant en place dans le moule (10).

3. Procédé selon la revendication précédente, dans lequel la capsule (6) de moulage comprend sur la portion (8) libre au moins une ouverture (9) pour la prise avec le matériau adapté à former le moule (10).

4. Procédé selon l'une quelconque des revendications précédentes, dans lequel le moule (10) est monobloc, et comprend une ouverture (13) pour le remplissage du moule (10) et pour l'extraction du modèle (1).

5. Procédé selon la revendication précédente, dans lequel un système (15) de préhension est fixé sur le modèle (1), le procédé comprenant, avant l'extraction du modèle (1) hors du moule (10), la mise en place du système (15) de préhension sur le modèle (1) et la prise du système (15) de préhension dans le matériau pour le modèle (1), l'extraction du modèle (1) hors du moule (10) comprenant la coopération entre le système (15) de préhension en prise avec le matériau du modèle dans le moule et un dispositif (19) d'extraction.

6. Procédé selon la revendication précédente, comprenant, après l'extraction du modèle (1) hors du moule (10), le retrait complet du système (15) de préhension hors du modèle (1).

7. Procédé selon la revendication 5, dans lequel le système (15) de préhension comprend un support (26) pour un aimant (29), le procédé comprenant après l'extraction du modèle (1) hors du moule (10) le retrait d'une partie seulement du système (15) de préhension, le support (26) pour l'aimant (29) restant en place sur le modèle (1).

8. Procédé selon l'une quelconque des revendications précédentes, dans lequel le modèle (1) comprend au moins un doigt d'une main.

9. Procédé selon l'une quelconque des revendications précédentes, dans lequel le modèle (1) comprend une main complète.

10. Procédé selon l'une quelconque des revendications précédentes, dans lequel le matériau du modèle comprend du silicone.

11. Moule (10) pour la fabrication d'un modèle d'au moins une partie de membre, le moule (10) comprenant une empreinte (11) de la au moins une partie de membre et au moins une capsule (6) de moulage d'ongle, la capsule (6) de moulage d'ongle comprenant au moins une portion (8) en prise dans le moule (10) et au moins une portion (7) en saillie à l'intérieur du moule (10), le moule (10) étant particulièrement destiné à la mise en œuvre du procédé de fabrication selon l'une quelconque des revendications précédentes.

12. Kit pour l'entraînement aux soins d'un membre et/ou des ongles, le kit comprenant au moins un modèle (1) obtenu par la mise en œuvre du procédé de fabrication selon l'une quelconque des revendications 1 à 10, le modèle (1) étant muni d'au moins une fente (3) et le kit comprenant au moins une capsule (14) d'ongles de forme et de dimension ajustées à celles de la fente (3).
